# EUROPEAN PATENT SPECIFICATION

(11) **EP 3 748 355 B1**
(45) Date of publication and mention of the grant of the patent: **22.03.2023**
(21) Application number: 20176981.7
(22) Date of filing: 28.05.2020
(51) Int. Cl.: G01N 33/02

(54) **A METHOD FOR FOOD PACKAGE ASSESSMENT, AND A SYSTEM THEREOF**
VERFAHREN ZUR BEWERTUNG VON LEBENSMITTELVERPACKUNGEN UND SYSTEM DAFÜR
PROCÉDÉ D'ÉVALUATION D'EMBALLAGES ALIMENTAIRES ET SYSTÈME ASSOCIÉ

(30) Priority: 05.06.2019 EP 19178434
(43) Date of publication of application: 09.12.2020
(73) Proprietor: Tetra Laval Holdings & Finance S.A., 1009 Pully (CH)
(72) Inventor: CORAZZARI, Gianni, 41032 Cavezzo (MO) (IT); Pellacani, Alex, 41034 Finale Emilia (MO) (IT); Rossi, Riccardo, 41043 Magreta (MO) (IT)
(74) Representative: Tetra Pak - Patent Attorneys SE

(56) References cited:
- CA-A1- 2 718 478

## Description

### Technical Field

The invention relates to the field of packaging technology. More particularly, it is related to food package assessment, such as food package integrity assessment.

### Background Art

Food safety is to be considered in every step of food production. Several quality assessment steps are typically included in a food production process to assure that there is no unwanted microorganisms in a food product being produced. In addition to the food production, packages in which the product is filled also need to comply with food safety standards in order to assure that the product can be consumed safely.

Different types of tests are made to assure food safety. In food production equipment, different types of sensors can be applied to make sure that the product is adequately heat treated or that the fat content is within pre-set intervals. Vision sensor systems may be used in order to assure that the packages are produced in accordance with pre-set conditions. For instance, vision sensors may be used for capturing image data depicting the packages, which image data can be processed such that it can be concluded whether or not a shape of the packages are within pre-set intervals, which in turn provides information about whether or not the packages has been produced without deviations.

Even though food safety parameters can be monitored continuously by using different kinds of sensors, there is still a need to do off line tests as well. For instance, the packages may be within pre-set intervals in terms of shape, but still have problems in terms of e.g. package integrity. To make sure that these packages are not delivered to consumers, sample packages can be taken from the packages and analyzed off line. In case it is identified that the sample packages are not safe to consume, packages from the same batch can be identified and recalled.

As described above, today there are processes and technologies in place to assure food safety. However, there is still room for improvement in terms of efficiency. For instance, in order to be sure that no packages that may contain harmful food product are consumed, significant numbers of packages are wasted. If the process for identifying food safety issues could be made more efficient, less packages would need to be wasted, which would be both environmentally and financially beneficial. Document CA2718478 A1 is an example of a system and method of providing product quality and safety that may be of interest for the present application.

### Summary

It is an object of the invention to at least partly overcome one or more of the above-identified limitations of the prior art. In particular, it is an object to provide a method and system in accordance with the independent claims for food package integrity assessment that are more time efficient than today.

According to a first aspect it is provided a method for food package assessment. The method comprises determining an identity associated to a device linked to an operator to perform one or several tasks related to the food package assessment, transmitting instructions related to the food package assessment to the device associated with the identity, receiving measurements from the device in response to the instructions, and determining food package assessment data based on the measurements.

The method further comprises determining locations associated to a plurality of devices associated with a plurality of identities, determining an instructions-associated location linked to the instructions, wherein the step of determining the identity linked to the operator identified to perform one or several tasks related to the food package assessment comprises identifying the device associated to the identity having a location closest to the instructions-associated location.

The method may further comprise receiving a package identity together with the measurements, wherein the package identity is determined by the device.

The method may further comprise determining a package location by using the device, receiving the package location together with the measurements, determining further instructions based on the measurements, determining a further identity linked to a further operator based on the package location, transmitting the further instructions to the further operator, receiving further measurements from the further operator, wherein the food package assessment may be based on the measurements and the further measurements.

The method may further comprise receiving conditions for surroundings based on the package location.

The step of determining the package location may comprise scanning a package associated to the package identity by using the device, determining a device location for the device during the step of scanning the package, and setting the package location to the device location.

The food package assessment may comprise incubation of packages.

The method may further comprise comparing the measurements from the device linked to the identity with other measurements from other devices linked to other identities, and if deviations are found, transmitting a notification to the device linked to the identity.

The method may further comprise analyzing the food package assessment data to identify a food safety risk, if the food safety risk is identified, transmitting a recall request, and/or stopping food production.

According to a second aspect it is provided a system for food package assessment. The system comprises a plurality of devices associated to identities and linked to operators, a server configured to determine an identity among the identities, linked to an operator identified to perform one or several tasks related to the food package assessment, and to transmit instructions related to the food package assessment to a device associated with the identity, and receive measurements from the device in response to the instructions, and determine food package assessment data based on the measurements.

The server is further configured to determine locations associated to a plurality of devices associated to a plurality of identities, and determine an instructions-associated location linked to the instructions, and to identify the device associated to the identity having a location closest to the instructions-associated location.

The server may further be configured to receive a package identity together with the measurements, and the plurality of devices may be configured to determine the package identity.

The server may be further configured to receive a package identity and a package location together with the measurements, wherein the plurality of devices may be configured to determine the package identity and the package location.

The server may be further configured to receive conditions for surroundings together with the measurements, and wherein the plurality of devices may further be configured to receive conditions for surroundings.

The plurality of devices may further be configured to scan a package associated to the package identity, determine a device location while scanning the package, and set the package location to the device location.

Still other objectives, features, aspects and advantages of the invention will appear from the following detailed description as well as from the drawings.

### Brief Description of the Drawings

Embodiments of the invention will now be described, by way of example, with reference to the accompanying schematic drawings, in which
Fig. 1 is a known system for assessing food package integrity.
Fig. 2 is a system according to the invention.
Fig. 3a and 3b is a flowchart illustrating a method for food package assessment.

### Detailed description

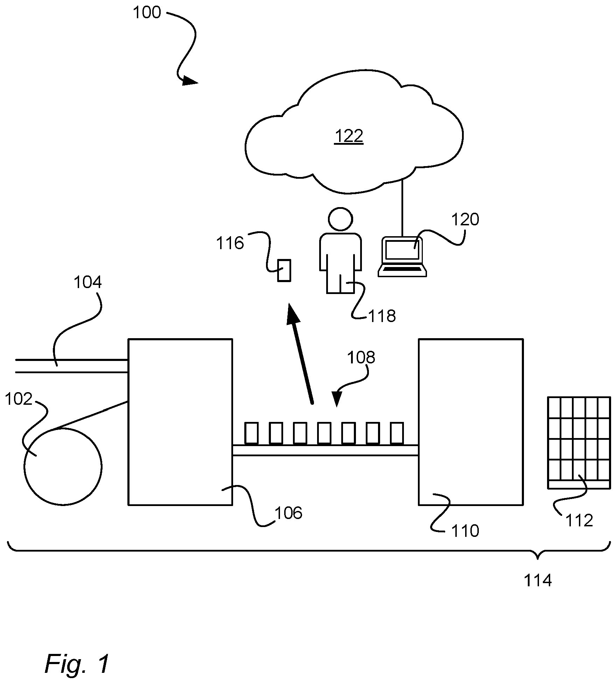

With reference to Fig. 1, a known system 100 for assessing food package integrity is illustrated. The known system 100 comprises a reel of packaging material 102 and a pipe 104 providing packaging material and food product, respectively, to a filling machine 106, also known as a packaging machine. In the particular example illustrated in Fig. 1, the packaging material is a carton-based packaging material and the filling machine 106 is a roll-fed filling machine. Other variants are blanks-fed filling machines or PET bottling machines.

The filling machine 106 is producing packages 108 filled with the food product, such as milk. After being produced, the packages 108 can be further handled by downstream equipment, such as card board packers, film wrappers, straw applicators, palletizers, etc. In the particular example illustrated, the packages 108 are fed to a palletizer 110 that is loading the packages onto a pallet 112.

Overall, pieces of equipment involved in packaging the food product, in this particular example the filling machine 106 and the palletizer 110, may be referred to as a packaging line 114.

To assess package integrity, i.e. that the packages 108 are closed such that no unwanted microorganisms or other external factors may spoil the food product, a sample package 116 may be captured from the packages 108 by an operator 118. After having received the sample package 116, the operator 118 performs a number of measurements on the sample package 116 and enters information into a computer 120. The computer 120 may be a stand-alone computer, but it may also be connected to other computers via a cloud service 122. In case the operator 118 finds that the sample package 116 does not meet pre-set quality conditions, the operator 118 may request that further actions are taken.

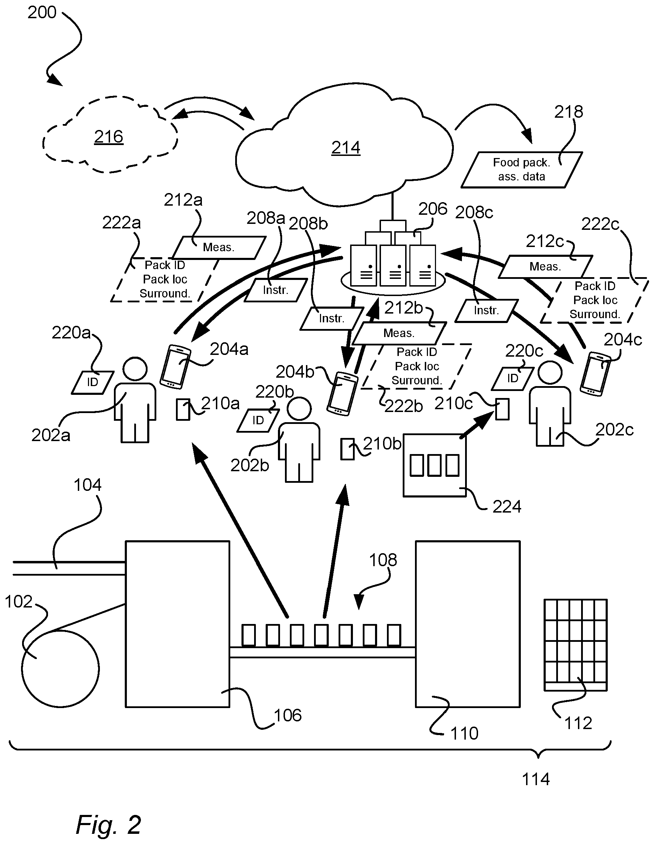

Fig. 2 illustrates a system 200 according to the present invention. As the known system 100 illustrated in Fig. 1, the system 200 comprises the packaging line 114 comprising the reel 102, the pipe 104, the filling machine 106, the packages 108, the palletizer and the pallet 112.

However, unlike the known system 100, the system 200 comprises a plurality of operators 202a, 202b, 202c, each having a device 204a, 204b, 204c, such as a mobile phone or tablet, communicatively connected to a server 206. Having such a set-up it is made possible to send instructions 208a, 208b,208c to the devices 204a, 204b, 204c from the server 206 whenever needed. Thus, instead of having a passive approach that is based on that the operator 118, as illustrated in Fig. 1, follows a scheme provided to him or her, the system 200 provides the possibility for an active approach in which the instructions 208a, 208b, 208c are provided to the operators 202a, 202b ,202c.

After having transmitted the instructions 208a, 208b, 208c, which may be provided to one or several of the operators 202a, 202b, 202c, the operators capture sample packages 210a, 210b, 210c and perform tasks assigned in the instructions 208a, 208b, 208c. Where to pick the sample packages 210a, 210b, 210c may be included in the instructions. Measurements 212a, 212b, 212c made on the sample packages 210a, 210b, 210c can be transferred from the devices 204a, 204b, 204c to the server 206.

The measurements 212a, 212b, 212c can be processed by the server 206 itself or by a cloud service 214, or a combination thereof. Optionally, additional data may be received from another cloud service 216 associated with another packaging line such that the measurements can be assessed more reliably. In a similar manner, data may be transmitted from the cloud service 214 associated to the server 206 to the other cloud service 216.

An output from the processing of the measurements is food packaging assessment data 216. This data may be a Boolean indicating only whether or not the packages 210a, 210b, 210c provide sufficient integrity or not, or it may contain more detailed data such that e.g. a root cause for insufficient integrity can be identified.

The devices 204a, 204b, 204c may be associated with identities 220a, 220b, 220c. In case the devices are mobile phones or tablets, the identities may be MAC addresses, but the identities may also be made specifically for this purpose. An advantage with having identities for the devices is that, if a specific operator is associated to a specific device, operator-specific deviations in measurements can be identified. As an effect, if it can be identified that the specific operator is not performing the measurements correctly, this can be identified and training may be provided such that in turn a reliability of the measurements can be improved.

Further, the devices 204a, 204b, 204c may be provided with cameras such that optical marks, such as QR codes, provided on the packages can be scanned. Since the optical marks may contain package identity information, it is possible not only to identify the operator performing the tasks resulting in the measurements, but also a package identity of the package being measured. The package identity 222a, 222b, 22c may be transmitted from the device together with the measurements. By knowing the package identity, the server 206 may capture additional information in the form of e.g. parameter settings used in the filling machine 106 when producing the package, which in turn provides for that an improved analysis of the measurements can be made.

Still further, the devices 204a, 204b, 204c are GPS-enabled or in any other way provided with means for determining their positions. By having the positions of the devices, it is possible to also determine where the measurements take place since the devices are used for performing the measurements. This especially holds true if the devices are used for scanning the packages to determine the package identities. In this way also the package location 222a, 222b, 222c may be transmitted together with the measurements.

Another effect of having position information for the devices 204a, 204b, 204c is that the instructions are sent to an operator being close to a location from which the packages are to be captured. For instance, if it can be determined that the operator is close to the palletizer 110 and the package to be measured is to be captured from the palletizer, the instructions may be sent to this operator.

Having the positions of the devices 204a, 204b, 204c also provides for that conditions for the surroundings 222a, 222b, 222c can be identified and transmitted together with the measurements. The conditions may be captured by having sensors, such as temperature sensors and humidity sensors, incorporated in the devices, but it is also possible for the devices 204a, 204b, 204c to indirectly determine the conditions by sending a request for the conditions together with the position data to external sensors, and receiving the conditions from the external sensors. The external sensors may e.g. be temperature sensors and/or humidity sensors provided in a facility housing the packaging line 114.

Identifying the conditions for the surroundings may be of relevance since temperature and/or humidity may affect the measurements, and the conditions for the surroundings may vary in different parts of the facility housing the packaging line. For instance, the packaging line 114 may be arranged such that the pipe 104 and the reel 102 is placed in a cold section, e.g. below 10 degrees Celsius, of the facility and the palletizer 110 is placed in a warm section, above 10 degrees Celsius, of the facility.

Even though illustrated that the package identity, the package location and the conditions for the surroundings are grouped, it should be understood that these can be handled separately as well.

As illustrated in Fig. 2, the sample packages 110a, 110b, 110c may not only be captured from the packaging line 114, but also from an incubator 224. By holding the sample packages for a pre-determined period of time under controlled conditions, reliable assessment of the package integrity can be made. Further, even though not illustrated, the sample packages may be captured from a ware house, placed at a different location than the packaging line, or in a food store, also placed elsewhere.

An advantage with the approach described above is that the assessment of packages can be made quicker, which provides for that food safety risks can be identified quicker, which in turn further reduces a risk that food product not safe to consume reaches consumers and also that the food production can be stopped such that a number of wasted packages can be reduced.

Still an advantage is that the assessment may be dynamic. For instance, in case a certain measurement is received it may be pre-programmed in the server 206 which instructions to transmit as a next step. The dynamic approach comes especially handy when being combined with several devices placed in different locations. For instance, in case the sample package held in the incubator 224 indicates that there may be a food safety risk, instructions may be sent to an operator placed in the ware house, placed in a different location, to perform measurements on packages kept in the ware house and coming from the same batch as the sample package held in the incubator.

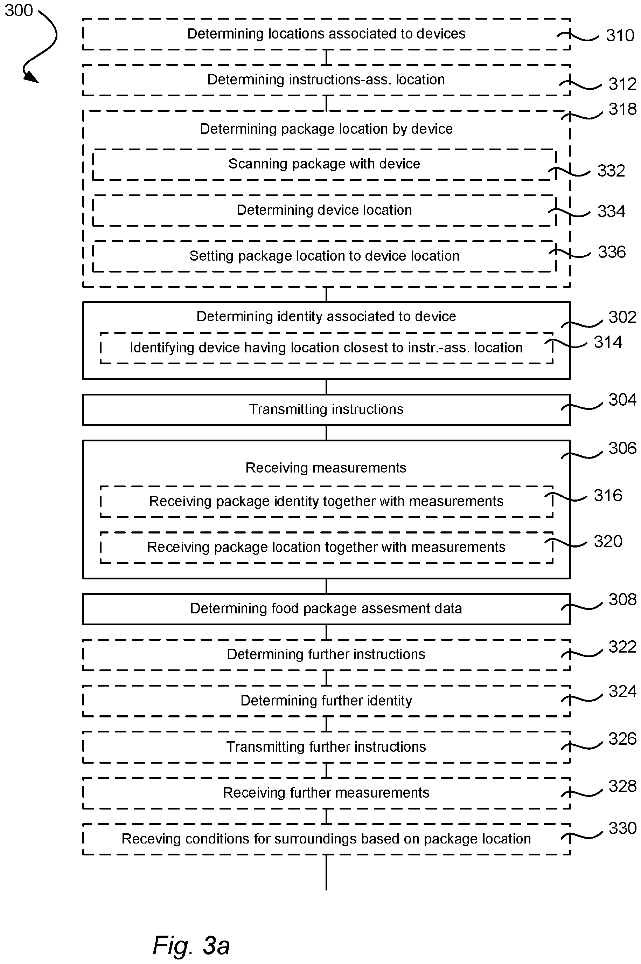

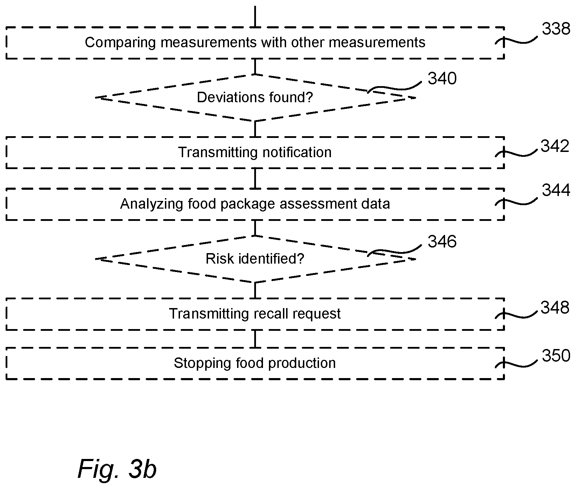

Fig. 3a and 3b is a flowchart 300 illustrating a method for food package assessment.

In a first step 302 an identity associated to the device 204a, 204b, 204c can be identified.

In a second step 304, the instructions 208a, 208b, 208c can be transmitted to the device.

In a third step 306, the measurements 212a, 212b, 212c from the device can be received.

In a fourth step 308, the food package assessment data 218 can be determined.

In a fifth step 310, locations associated to a plurality of devices are determined. In a sixth step 312, an instructions-associated location is determined. In a seventh step 314, that may be part of the first step 302, a device having a location closest to the instructions-associated location is identified. In other words, if the instructions are to be performed in a specific location, the device closest to this specific location is identified and instructions are transmitted to this device.

In an eighth step 316, a package identity may be received together with the measurements.

In a ninth step 318, a package location may be determined by the device.

In a tenth step 320, the package location may be received together with the measurements.

In an eleventh step 322, further instructions may be determined based on the measurements.

In a twelfth step 324, a further identity linked to a further operator may be determined based on the package location,

In a thirteenth step 326, the further instructions may be transmitted to the further operator.

In a fourteenth step 328, further measurements may be received from the further operator.

In a fifteenth step 330, conditions for surroundings determined based on the package location may be received.

The ninth step 318 may comprise a first sub-step 332 of scanning the package with the device, a second sub-step 334 of determining the device location during the step of scanning, and a third sub-step 336 of setting the package location to the device location.

In a sixteenth step 338, the measurements from the device linked to the identity can be compared with other measurements from other devices linked to other identities. If deviations are found 340, in a seventeenth step 342 a notification to the device linked to the identity may be transmitted.

In an eighteenth step 344, the food package assessment data 218 can be analyzed to identify a food safety risk. If the food safety risk is identified 346, in a nineteenth step 348 a recall request can be transmitted, and/or, in a twentieth step 350 the food production can be stopped.

Even though illustrated and described in a certain order it should be understood that the steps can be made in other orders as well.

From the description above follows that, although various embodiments of the invention have been described and shown, the invention is not restricted thereto, but may also be embodied in other ways within the scope of the subject-matter defined in the following claims.

## Claims

1. A method (300) for food package assessment, said method comprising
determining (302) an identity (220a, 220b, 220c) associated to a device (204a, 204b, 204c) linked to an operator (202a, 202b, 202c) to perform one or several tasks related to the food package assessment,
transmitting (304) instructions (208a, 208b, 208c) related to the food package assessment to the device (204a, 204b, 204c) associated with the identity (220a, 220b, 220c),
receiving (306) measurements (212a, 212b, 212c) from the device (204a, 204b, 204c) in response to the instructions (208a, 208b, 208c), and
determining (308) food package assessment data (218) based on the measurements (212a, 212b, 212c),
determining (310) locations associated to a plurality of devices (204a, 204b, 204c) associated with a plurality of identities (220a, 220b, 220c),
determining (312) an instructions-associated location linked to the instructions (208a, 208b, 208c),
wherein the step of determining (302) the identity linked to the operator identified to perform one or several tasks related to the food package assessment comprises
identifying (314) the device associated to the identity having a location closest to the instructions-associated location.

2. The method according to claim 1, further comprising
receiving (316) a package identity (222a, 222b, 222c) together with the measurements (212a, 212b, 212c), wherein the package identity is determined by the device (204a, 204b, 204c).

3. The method according to claim 2, further comprising
determining (318) a package location by using the device (204a, 204b, 204c),
receiving (320) the package location together with the measurements (212a, 212b, 212c),
determining (322) further instructions based on the measurements,
determining (324) a further identity linked to a further operator based on the package location,
transmitting (326) the further instructions to the further operator,
receiving (328) further measurements from the further operator,
wherein the food package assessment is based on the measurements and the further measurements.

4. The method according to claim 3, further comprising
receiving (330) conditions for surroundings based on the package location.

5. The method according to claim 3 or 4, wherein the step of determining (318) the package location comprises
scanning (332) a package associated to the package identity by using the device (204a, 204b, 204c),
determining (334) a device location for the device during the step of scanning the package (210a, 210b, 210c), and
setting (336) the package location to the device location.

6. The method according to any one of the preceding claims, wherein the food package assessment comprises incubation of packages.

7. The method according to any one of the preceding claims, further comprising
comparing (338) the measurements from the device linked to the identity with other measurements from other devices linked to other identities, and
if deviations are found (340),
transmitting (342) a notification to the device linked to the identity.

8. The method according to any one of the preceding claims, further comprising
analyzing (344) the food package assessment data (218) to identify a food safety risk,
if the food safety risk is identified (346),
transmitting (348) a recall request, and/or
stopping (350) food production.

9. A system (200) for food package assessment, said system (200) comprising
a plurality of devices (204a, 204b, 204c) associated to identities (220a, 220b, 220c) and linked to operators (202a, 202b, 202c),
a server (206) configured to:
determine an identity among the identities (220a, 200b, 200c), linked to an operator identified to perform one or several tasks related to the food package assessment,
transmit instructions (208a, 208b, 208c) related to the food package assessment to a device associated with the identity,
receive measurements (212a, 212b, 212c) from the device in response to the instructions,
determine food package assessment data (218) based on the measurements, determine locations associated to a plurality of devices (204a, 204b, 204c) associated to a plurality of identities (220a, 220,b, 220c),
determine an instructions-associated location linked to the instructions (208a, 208b, 208c), and
identify the device associated to the identity having a location closest to the instructions-associated location.

10. The system according to claim 9, wherein the server (206) is further configured to receive a package identity (222a, 222b, 222c) together with the measurements (212a, 212b, 212c), and
the plurality of devices (204a, 204b, 204c) are configured to determine the package identity.

11. The system according to any one of the claims 9 or 10, wherein the server (206) is further configured to receive a package identity and a package location (222a, 222b, 222c) together with the measurements (212a, 212b, 212c),
wherein the plurality of devices (204a, 204b, 204c) are configured to determine the package identity and the package location.

12. The system according to claim 11, wherein the server (206) is further configured to receive conditions for surroundings (222a, 222b, 222c) together with the measurements (212a, 212b, 212c), and
wherein the plurality of devices (204a, 204b, 204c) are further configured to receive conditions for surroundings (222a, 22b, 222c).

13. The system according to any one of the claims 9 to 12, wherein the plurality of devices (204a, 204b, 204c) are further configured to scan a package associated to the package identity, determine a device location while scanning the package, and set the package location to the device location.

## Patentansprüche

1. Verfahren (300) zur Bewertung von Lebensmittelverpackungen, wobei das Verfahren Folgendes umfasst:
Bestimmen (302) einer Identität (220a, 220b, 220c), die einer Vorrichtung (204a, 204b, 204c) zugeordnet ist, die mit einem Arbeiter (202a, 202b, 202c) zum Ausführen einer oder mehrerer Aufgaben im Zusammenhang mit der Bewertung der Lebensmittelverpackungen verbunden ist,
Senden (304) von Anweisungen (208a, 208b, 208c) im Zusammenhang mit der Bewertung der Lebensmittelverpackungen an die Vorrichtung (204a, 204b, 204c), die der Identität (220a, 220b, 220c) zugeordnet ist,
Empfangen (306) von Messungen (212a, 212b, 212c) von der Vorrichtung (204a, 204b, 204c) als Reaktion auf die Anweisungen (208a, 208b, 208c) und
Bestimmen (308) von die Bewertung der Lebensmittelverpackung betreffenden Daten (218) basierend auf den Messungen (212a, 212b, 212c),
Bestimmen (310) von Standorten, die mehreren Vorrichtungen (204a, 204b, 204c), die mehreren Identitäten (220a, 220b, 220c) zugeordnet sind, zugeordnet sind,
Bestimmen (312) eines Anweisungen zugeordneten Standorts, der mit den Anweisungen (208a, 208b, 208c) verbunden ist,
wobei der Schritt des Bestimmens (302) der Identität, die mit dem Arbeiter, der zum Ausführen einer oder mehrerer Aufgaben im Zusammenhang mit der Bewertung der Lebensmittelverpackungen ausersehen wurde, verbunden ist, Folgendes umfasst:
Ermitteln (314) der Vorrichtung, die der Identität mit einem Standort, der dem Anweisungen zugeordneten Standort am nächsten ist, zugeordnet ist.

2. Verfahren nach Anspruch 1, ferner umfassend:
Empfangen (316) einer Verpackungsidentität (222a, 222b, 222c) zusammen mit den Messungen (212a, 212b, 212c), wobei die Verpackungsidentität durch die Vorrichtung (204a, 204b, 204c) bestimmt wird.

3. Verfahren nach Anspruch 2, ferner umfassend:
Bestimmen (318) eines Verpackungsstandorts unter Verwendung der Vorrichtung (204a, 204b, 204c),
Empfangen (320) des Verpackungsstandorts zusammen mit den Messungen (212a, 212b, 212c),
Bestimmen (322) weiterer Anweisungen basierend auf den Messungen,
Bestimmen (324) einer weiteren Identität, die mit einem weiteren Arbeiter verbunden ist, basierend auf dem Verpackungsstandort,
Senden (326) der weiteren Anweisungen an den weiteren Arbeiter,
Empfangen (328) weiterer Messungen von dem weiteren Arbeiter,
wobei die Bewertung der Lebensmittelverpackungen auf den Messungen und den weiteren Messungen basiert.

4. Verfahren nach Anspruch 3, ferner umfassend:
Empfangen (330) von Umgebungsbedingungen basierend auf dem Verpackungsstandort.

5. Verfahren nach Anspruch 3 oder 4, wobei der Schritt des Bestimmens (318) des Verpackungsstandorts Folgendes umfasst:
Scannen (332) einer Verpackung, die der Verpackungsidentität zugeordnet ist, unter Verwendung der Vorrichtung (204a, 204b, 204c),
Bestimmen (334) eines Vorrichtungsstandorts für die Vorrichtung während des Schritts des Scannens der Verpackung (210a, 210b, 210c) und
Einstellen (336) des Verpackungsstandorts auf den Vorrichtungsstandort.

6. Verfahren nach einem der vorangehenden Ansprüche, wobei die Bewertung der Lebensmittelverpackungen eine Inkubation von Verpackungen umfasst.

7. Verfahren nach einem der vorangehenden Ansprüche, ferner umfassend:
Vergleichen (338) der Messungen von der Vorrichtung, die mit der Identität verbunden ist, mit anderen Messungen von anderen Vorrichtungen, die mit anderen Identitäten verbunden sind, und
wenn Abweichungen festgestellt werden (340),
Senden (342) einer Benachrichtigung an die Vorrichtung, die mit der Identität verbunden ist.

8. Verfahren nach einem der vorangehenden Ansprüche, ferner umfassend:
Analysieren (344) der die Bewertung der Lebensmittelverpackungen betreffenden Daten (218) zum Ermitteln eines Lebensmittelsicherheitsrisikos,
wenn das Lebensmittelsicherheitsrisiko ermittelt wird (346),
Senden (348) einer Rückrufaufforderung und/oder
Stoppen (350) der Lebensmittelproduktion.

9. System (200) zur Bewertung von Lebensmittelverpackungen, wobei das System (200) Folgendes umfasst:
mehrere Vorrichtungen (204a, 204b, 204c), die Identitäten (220a, 220b, 220c) zugeordnet und mit Arbeitern (202a, 202b, 202c) verbunden sind,
einen Server (206), der für Folgendes ausgestaltet ist:
Bestimmen - aus den Identitäten (220a, 200b, 200c) - einer Identität, die mit einem Arbeiter, der zum Ausführen einer oder mehrerer Aufgaben im Zusammenhang mit der Bewertung der Lebensmittelverpackungen ausersehen wurde, verbunden ist,
Senden von Anweisungen (208a, 208b, 208c) im Zusammenhang mit der Bewertung der Lebensmittelverpackungen an eine Vorrichtung, die der Identität zugeordnet ist,
Empfangen von Messungen (212a, 212b, 212c) von der Vorrichtung als Reaktion auf die Anweisungen,
Bestimmen von die Bewertung der Lebensmittelverpackungen betreffenden Daten (218) basierend auf den Messungen,
Bestimmen von Standorten, die mehreren Vorrichtungen (204a, 204b, 204c), die mehreren Identitäten (220a, 220b, 220c) zugeordnet sind, zugeordnet sind,
Bestimmen eines Anweisungen zugeordneten Standorts, der mit den Anweisungen (208a, 208b, 208c) verbunden ist, und
Ermitteln der Vorrichtung, die der Identität mit einem Standort, der dem Anweisungen zugeordneten Standort am nächsten ist, zugeordnet ist.

10. System nach Anspruch 9, wobei der Server (206) ferner dazu ausgestaltet ist, zusammen mit den Messungen (212a, 212b, 212c) eine Verpackungsidentität (222a, 222b, 222c) zu empfangen, und
die mehreren Vorrichtungen (204a, 204b, 204c) dazu ausgestaltet sind, die Verpackungsidentität zu bestimmen.

11. System nach einem der Ansprüche 9 oder 10, wobei der Server (206) ferner dazu ausgestaltet ist, zusammen mit den Messungen (212a, 212b, 212c) eine Verpackungsidentität und einen Verpackungsstandort (222a, 222b, 222c) zu empfangen,
wobei die mehreren Vorrichtungen (204a, 204b, 204c) dazu ausgestaltet sind, die Verpackungsidentität und den Verpackungsstandort zu bestimmen.

12. System nach Anspruch 11, wobei der Server (206) ferner dazu ausgestaltet ist, zusammen mit den Messungen (212a, 212b, 212c) Umgebungsbedingungen (222a, 222b, 222c) zu empfangen, und
wobei die mehreren Vorrichtungen (204a, 204b, 204c) ferner dazu ausgestaltet sind, Umgebungsbedingungen (222a, 22b, 222c) zu empfangen.

13. System nach einem der Ansprüche 9 bis 12, wobei die mehreren Vorrichtungen (204a, 204b, 204c) ferner dazu ausgestaltet sind, eine Verpackung, die der Verpackungsidentität zugeordnet ist, zu scannen, während des Scannens der Verpackung einen Vorrichtungsstandort zu bestimmen und den Verpackungsstandort auf den Vorrichtungsstandort einzustellen.

## Revendications

1. Procédé (300) d'évaluation d'emballage alimentaire, ledit procédé comprenant :
la détermination (302) d'une identité (220a, 220b, 220c) associée à un dispositif (204a, 204b, 204c) lié à un opérateur (202a, 202b, 202c) pour réaliser une ou plusieurs tâches liées à l'évaluation d'emballage alimentaire,
la transmission (304) d'instructions (208a, 208b, 208c) liées à l'évaluation d'emballage alimentaire au dispositif (204a, 204b, 204c) associé à l'identité (220a, 220b, 220c),
la réception (306) de mesures (212a, 212b, 212c) en provenance du dispositif (204a, 204b, 204c) en réponse aux instructions (208a, 208b, 208c), et
la détermination (308) de données d'évaluation d'emballage alimentaire (218) sur la base des mesures (212a, 212b, 212c),
la détermination (310) d'emplacements associés à une pluralité de dispositifs (204a, 204b, 204c) associés à une pluralité d'identités (220a, 220b, 220c),
la détermination (312) d'un emplacement associé à des instructions liées aux instructions (208a, 208b, 208c),
l'étape de détermination (302) de l'identité liée à l'opérateur identifié pour réaliser une ou plusieurs tâches liées à l'évaluation d'emballage alimentaire comprenant :
l'identification (314) du dispositif associé à l'identité ayant un emplacement le plus proche de l'emplacement associé aux instructions.

2. Procédé selon la revendication 1, comprenant en outre :
la réception (316) d'une identité d'emballage (222a, 222b, 222c) conjointement avec les mesures (212a, 212b, 212c), l'identité d'emballage étant déterminée par le dispositif (204a, 204b, 204c).

3. Procédé selon la revendication 2, comprenant en outre :
la détermination (318) d'un emplacement d'emballage en utilisant le dispositif (204a, 204b, 204c),
la réception (320) de l'emplacement d'emballage conjointement avec les mesures (212a, 212b, 212c),
la détermination (322) d'instructions supplémentaires sur la base des mesures,
la détermination (324) d'une identité supplémentaire liée à un opérateur supplémentaire sur la base de l'emplacement d'emballage,
la transmission (326) des instructions supplémentaires à l'opérateur supplémentaire,
la réception (328) de mesures supplémentaires en provenance de l'opérateur supplémentaire,
l'évaluation d'emballage alimentaire étant sur la base des mesures et des mesures supplémentaires.

4. Procédé selon la revendication 3, comprenant en outre la réception (330) de conditions d'environnement sur la base de l'emplacement d'emballage.

5. Procédé selon la revendication 3 ou 4, l'étape de détermination (318) de l'emplacement d'emballage comprenant :
le balayage (332) d'un emballage associé à l'identité d'emballage en utilisant le dispositif (204a, 204b, 204c),
la détermination (334) d'un emplacement de dispositif pour le dispositif pendant l'étape de balayage de l'emballage (210a, 210b, 210c), et
le réglage (336) de l'emplacement d'emballage sur l'emplacement du dispositif.

6. Procédé selon l'une quelconque des revendications précédentes, l'évaluation d'emballage alimentaire comprenant l'incubation des emballages.

7. Procédé selon l'une quelconque des revendications précédentes, comprenant en outre :
la comparaison (338) des mesures du dispositif lié à l'identité avec des mesures supplémentaires de dispositifs supplémentaires liés à des identités supplémentaires, et
si des écarts sont constatés (340),
la transmission (342) d'une notification au dispositif lié à l'identité.

8. Procédé selon l'une quelconque des revendications précédentes, comprenant en outre :
l'analyse (344) des données d'évaluation d'emballage alimentaire (218) pour identifier un risque de sécurité alimentaire,
si le risque de sécurité alimentaire est identifié (346),
la transmission (348) d'une demande de rappel, et/ou
l'arrêt (350) de la production d'aliments.

9. Système (200) d'évaluation d'emballage alimentaire, ledit système (200) comprenant :
une pluralité de dispositifs (204a, 204b, 204c) associés à des identités (220a, 220b, 220c) et liés à des opérateurs (202a, 202b, 202c),
un serveur (206) configuré pour :
déterminer une identité parmi les identités (220a, 200b, 200c), liée à un opérateur identifié pour réaliser une ou plusieurs tâches liées à l'évaluation d'emballage alimentaire,
transmettre des instructions (208a, 208b, 208c) liées à l'évaluation d'emballage alimentaire à un dispositif associé à l'identité,
recevoir des mesures (212a, 212b, 212c) du dispositif en réponse aux instructions,
déterminer des données d'évaluation d'emballage alimentaire (218) sur la base des mesures,
déterminer des emplacements associés à une pluralité de dispositifs (204a, 204b, 204c) associés à une pluralité d'identités (220a, 220b, 220c),
déterminer un emplacement associé à des instructions liées aux instructions (208a, 208b, 208c), et
identifier le dispositif associé à l'identité ayant un emplacement le plus proche de l'emplacement associé aux instructions.

10. Système selon la revendication 9, le serveur (206) étant en outre configuré pour recevoir une identité d'emballage (222a, 222b, 222c) conjointement avec les mesures (212a, 212b, 212c), et
la pluralité de dispositifs (204a, 204b, 204c) étant configurée pour déterminer l'identité d'emballage.

11. Système selon l'une quelconque des revendications 9 ou 10, le serveur (206) étant en outre configuré pour recevoir une identité d'emballage et un emplacement d'emballage (222a, 222b, 222c) conjointement avec les mesures (212a, 212b, 212c),
la pluralité de dispositifs (204a, 204b, 204c) étant configurés pour déterminer l'identité d'emballage et l'emplacement d'emballage.

12. Système selon la revendication 11, le serveur (206) étant en outre configuré pour recevoir des conditions d'environnement (222a, 222b, 222c) conjointement avec les mesures (212a, 212b, 212c), et
la pluralité de dispositifs (204a, 204b, 204c) étant en outre configurés pour recevoir des conditions d'environnement (222a, 22b, 222c).

13. Système selon l'une quelconque des revendications 9 à 12, la pluralité de dispositifs (204a, 204b, 204c) étant en outre configurés pour balayer un emballage associé à l'identité d'emballage, déterminer un emplacement de dispositif tout en balayant l'emballage, et régler l'emplacement d'emballage sur l'emplacement du dispositif.
